# EUROPÄISCHE PATENTSCHRIFT

(11) **EP 2 091 871 B1**
(45) Veröffentlichungstag und Bekanntmachung des Hinweises auf die Patenterteilung: **12.05.2010**
(21) Anmeldenummer: 07786600.2
(22) Anmeldetag: 07.08.2007
(51) Int. Cl.: C02F 1/32

(54) **VERFAHREN ZUR ÜBERWACHUNG EINER MEHRZAHL VON ELEKTRISCHEN LEUCHTKÖRPERN UND VORRICHTUNG ZUR DESINFEKTION EINER SUBSTANZ MITTELS ULTRAVIOLETTER STRAHLUNG**
METHOD FOR MONITORING A PLURALITY OF ELECTRICAL LUMINOUS ELEMENTS AND DEVICE FOR DISINFECTING A SUBSTANCE BY MEANS OF ULTAVIOLET RADIATION
PROCÉDÉ DE SURVEILLANCE DE MULTIPLES CORPS LUMINEUX ÉLECTRIQUES ET DISPOSITIF DE DÉSINFECTION D'UNE SUBSTANCE AU MOYEN D'UN RAYONNEMENT ULTRAVIOLET

(30) Priorität: 23.10.2006 DE 102006050276
(43) Veröffentlichungstag der Anmeldung: 26.08.2009
(73) Patentinhaber: Wedeco AG, 32051 Herford (DE)
(72) Erfinder: LOESENBECK, Jan Boris, 33615 Bielefeld (DE)
(74) Vertreter: Lenzing Gerber Stute
(86) Internationale Anmeldenummer: PCT/EP2007/006970
(87) Internationale Veröffentlichungsnummer: WO 2008/049476

(56) Entgegenhaltungen:
- EP-A- 0 608 061
- WO-A-2005/019782
- DE-A1- 2 825 672
- US-A- 4 471 225
- US-A- 5 368 826

## Beschreibung

Die Erfindung betrifft ein Verfahren zur Überwachung einer Mehrzahl von elektrischen Leuchtkörpern sowie eine Vorrichtung zur Desinfektion einer Substanz mittels ultravioletter Strahlung nach dem Oberbegriff des Anspruchs 19.

Im Bereich der Desinfektion von Wasser, z. B. Trinkwasser, Abwasser, Schwimmbadwasser oder Ähnliches, sind Installationen bekannt, bei denen eine Mehrzahl von baugleichen UV-Gasentladungsröhren gleichzeitig betrieben werden. Je nach Größe der Anlage handelt es sich dabei zwischen einigen wenigen Röhren bis zu über 10.000 Röhren. Letztgenannte Großanlagen werden z. B. verwendet, um für die Klärung von Abwasser eingesetzte Mikroorganismen nach dem entsprechenden Klärschritt abzutöten. Die verwendeten UV-Röhren sind kostenintensiv und es ist daher gewünscht, diese in ihrer Lebensdauer zu optimieren. Andererseits ist es oft nicht zielführend, beim Betrieb einer solchen Anlage bis zum völligen Ausfall einer Röhre zu warten, da ihre Leistungsfähigkeit aufgrund einer Vielzahl möglicher Effekte bereits zuvor nachlässt. Gerade im Bereich der Desinfektion und Abtötung von Mikroorganismen muss zudem die volle Funktionsfähigkeit der Anlage jederzeit unbedingt gewährleistet sein.

Die US-A-5368826 offenbart ein UV-Desinfektionssystem insbesondere zur Desinfektion von Abwasser mit mehreren, in Reihe angeordneten UV-Lampen. Eine Kontrolleinheit empfängt und speichert Daten von jeder Lampe und überwacht den Betrieb jeder Lampe; die gemessenen Daten einer Lampe werden mit vorab festgelegten Soll-Werten verglichen.

Es hat sich gezeigt, dass die aktuellen Betriebsparameter eines Leuchtkörpers insbesondere bei Einsatz in einer Anlage zur Wasserdesinfektion keinen sehr zuverlässigen Anhaltspunkt für den Funktionszustand des Leuchtkörpers geben. Dies ist u. a. durch eine recht große Schwankungsbreite dieser Betriebsparameter in Abhängigkeit von äußeren Einflüssen wie etwa der Wassertemperatur, der Fliessgeschwindigkeit des Wassers u.a. bedingt.

Es ist die Aufgabe der Erfindung, ein Verfahren zur Überwachung einer Mehrzahl von zumindest drei baugleichen elektrischen Leuchtkörpern sowie eine Vorrichtung nach dem Oberbegriff des Anspruchs 19 anzugeben, bei denen die Ausnutzung der Lebensdauer der einzelnen Leuchtkörper individuell optimiert ist.

Diese Aufgabe wird für ein eingangs genanntes Verfahren mit den Merkmalen des Anspruchs 1 gelöst. Durch den Vergleich eines Parameters eines einzelnen der Leuchtkörper mit einem Referenzwert, der aus zumindest einigen der Parameter der anderen Leuchtkörper gebildet ist, lässt sich eine Aussage über den Funktionszustand des Leuchtkörpers gewinnen, die weitgehend unabhängig von äußeren Einflüssen ist, sofern diese Einflüsse im wesentlichen auf sämtliche Leuchtkörper gleich wirken. Insbesondere betrifft dies die Betriebstemperatur der Leuchtkörper, die von äußeren Bedingungen beeinflusst ist. Weicht einer der Leuchtkörper mit seinem Parameter von dem durch eine Menge von Leuchtkörpern gebildeten Referenzwert ausreichend deutlich ab, so wird ein entsprechendes Signal generiert. Dieses Signal kann zu einem unmittelbaren Austausch des Leuchtkörpers führen. Je nach Größe der Anlage und gegebenenfalls weiteren Informationen kann es aber auch vorgesehen sein, dass zunächst einige Signale gesammelt werden oder weitere Kontrollroutinen eingeleitet werden. Bei ausreichender Anzahl der Leuchtkörper und ausreichender Streuung der individuellen Lebensdauer sowie entsprechend häufiger Wiederholung der Verfahrensschritte ist die Wahrscheinlichkeit vernachlässigbar, dass gleich mehrere Leuchtkörper eine entsprechend große Abweichung ihres Parameters zeigen, so dass die Generierung eines Fehlsignals unwahrscheinlich ist.

In bevorzugter Ausführung ist der Referenzwert auf einfache Weise ein Mittelwert der Parameter.

Der Referenzwert kann insbesondere aus sämtlichen der ausgelesenen Parameter gebildet werden. Bei ausreichend großer Anzahl von Leuchtkörpern, die sich zumindest mehrheitlich in einem guten Betriebszustand befinden, wird auf diese Weise ein optimaler mittlerer Betriebswert der Leuchtkörper statistisch angenähert. Insbesondere im Fall von wenigen Leuchtkörpern kann jedoch das Abweichen eines Leuchtkörpers den Referenzwert bereits maßgeblich beeinflussen. In diesem Fall kann es bevorzugt vorgesehen sein, dass der Referenzwert jeweils ohne Einbeziehung des in Schritt c. mit dem Referenzwert zu vergleichenden Parameters gebildet wird.

Allgemein vorteilhaft werden die Schritte b. bis e insbesondere in gleichen Zeitabständen wiederholt. Die Wiederholdauer ist dabei vorteilhaft erheblich kleiner als die mittlere Lebensdauer eines Leuchtkörpers und insbesondere vorteilhaft kleiner als eine mittlere Abweichung der Lebensdauern verschiedener Leuchtkörper.

In bevorzugter Weiterbildung wird der Parameter für jeden der Leuchtkörper über einen Zeitraum mehrfach ausgelesen und gespeichert, wobei ein zeitlicher Verlauf der Parameter und des Referenzwertes ermittelbar ist. Hierdurch ist insbesondere eine Änderungsgeschwindigkeit des Parameters vorteilhaft ermittelbar, was unter Umständen eine wichtige Aussage liefern kann. So kommt es zum Beispiel bei UV-Gasentladungsröhren, die Amalgam enthalten, gelegentlich zum Abtropfen angehäuften A-malgams, was eine sehr kurzzeitige Änderung der Betriebsparameter der Röhre bewirkt. Die absolute Änderung des fraglichen Parameters kann dabei durchaus im tolerablen Bereich liegen, wobei die hohe Änderungsgeschwindigkeit des Signals diesen Leuchtkörper dennoch als einen zumindest im Rahmen einer nächsten Routinewartung auszutauschenden Leuchtkörper identifiziert. Bei einem anderen Beispiel kann der Zeitraum im Bereich der Einstellung eines thermischen Gleichgewichts nach einem Einschalten der Anlage liegen. Normal funktionierende Leuchtkörper haben jeweils eine ähnliche Zeitdauer zur Erreichung des thermischen Gleichgewichts, wobei diese Zeitdauer erheblich von den Umwelteinflüssen abhängen kann. Leuchtkörper, die beispielsweise eine Bedampfung und somit Verringerung ihrer nach außen abgegebenen Strahlungsleistung aufweisen, weisen regelmäßig eine signifikant abweichende Zeitdauer zur Erreichung ihres thermischen Gleichgewichts auf. Hiervon abgesehen kann ein solcher Leuchtkörper mit seinen weiteren Betriebsparametern im Gleichgewichtszustand durchaus unauffällig erscheinen. Weiterhin könnte ein Kurzzeitverhalten des Leuchtkörpers ausgemessen und mit dem aus den anderen Leuchtkörpern gebildeten Referenzwert verglichen werden. Ein solches Kurzzeitverhalten könnte z. B. in einer Sprungantwort von Betriebsparametern auf beispielsweise eine instantane Änderung der Versorgungsspannung sein. Deutlich von dem referenzwert abweichende Sprungantworten können ein Frühindikator zur Erkennung eines schadhaften oder sich dem Ende seiner Lebensdauer annähenden Leuchtkörpers sein. Grundsätzlich ist in unter den Begriffen "Parameter" und "Referenzwert" des erfindungsgemäßen Verfahren auch differenzierte Auswertung einer solchen Antwortkurve oder allgemein einer über einen Zeitraum akquirierten Menge von Werten zu verstehen.

In einer bevorzugten Ausführungsform ist das Versorgungssignal des Vorschaltgerätes in Abhängigkeit von einem Sollwert geregelt. Gängige UV-Gasentladungsröhren werden häufig auf diese Art betrieben. Beispielsweise kann der Leuchtkörper auf eine konstante Stromstärke als Sollwert geregelt werden, wobei das Versorgungssignal bzw. eine Versorgungsspannung die zu regelnde Größe ist. Vorteilhaft ergibt sich dabei der Parameter des Leuchtkörpers aus dem an ihm anliegenden Versorgungssignal.
Auf einfache Weise ist der Parameter eine effektive Versorgungsspannung des Leuchtkörpers. Bei einer einfach realisierbaren und zuverlässigen Ausführung des erfindungsgemäßen Verfahrens ist der Referenzwert ein Mittelwert der effektiven Versorgungsspannungen der Leuchtkörper, wobei die vorgegebene Abweichung nicht mehr als etwa 10 %, insbesondere etwa 5 % von dem Mittelwert ist.

In besonders bevorzugter Ausführung ist der Sollwert eine effektive Stromstärke eines oder mehrerer der Leuchtkörper. Alternativ kann der Sollwert auch eine elektrische Leistung eines oder mehrerer der Leuchtkörper sein.

In einer günstig realisierbaren Ausführungsform ist das Versorgungssignal über eine Pulsweitenmodulation geregelt. Bevorzugt ist dabei über ein Tastverhältnis des Versorgungssignals eine effektive Versorgungsspannung einstellbar, wobei das Versorgungssignal insbesondere eine Rechteckspannung von konstanter Amplitude ist. Eine derartige Steuerung des Versorgungssignals ist auf besonders einfache und kostengünstige Weise mittels Digitalelektronik in Vorschaltgeräten realisierbar.

In bevorzugter Ausführung sind die Leuchtkörper jeweils Gasentladungsröhren. Gasentladungsröhren sind kostspielig und zeigen relativ komplexes Verhalten über ihre Lebensdauer. Sie sind zudem hinsichtlich ihrer Betriebsparameter recht empfindlich gegenüber äußeren Einflüssen wie etwa der Umgebungstemperatur, so dass das erfindungsgemäße Verfahren in besonderer Weise mit Gasentladungsröhren vorteilhaft kombinierbar ist.

Durch die Implementierung des erfindungsgemäßen Verfahrens lassen sich Betriebskosten und Betriebssicherheit besonders wirksam optimieren, wenn die Mehrzahl von Leuchtkörpern zumindest 10, insbesondere zumindest 100 Leuchtkörper umfasst. Grundsätzlich ist das Verfahren aber bereits bei drei separaten baugleichen Leuchtkörpern anwendbar, da unter der Annahme, dass zwei der drei Leuchtkörper in einem Sollzustand befindlich sind, die Abweichung des dritten Leuchtkörpers eindeutig feststellbar ist.

In vorteilhafter Ausführung ist jedem der Leuchtkörper ein Vorschaltgerät im Sinne eines unabhängigen Versorgungssignals zugeordnet. Grundsätzlich kann auch ein Vorschaltgerät sämtliche oder eine Untergruppe der Mehrzahl der Leuchtkorper betreiben. Z. B. kann dann ein konstanter Gesamtstrom der durch das Vorschaltgerät betriebenen Leuchtkörper eingeregelt werden. Um in solcher Anordnung Parameter individueller Leuchtkörper auslesen zu können, ist regelmäßig eine individuelle Messung beispielsweise der Versorgungsspannung der einzelnen Leuchtkörper erforderlich. Falls jedem Leuchtkörper ein separates Vorschaltgerät im Sinne einer separierten Versorgungsspannung zugeordnet ist, so kann die Versorgungsspannung oder auch ein anderer Betriebsparameter unmittelbar aus dem Vorschaltgerät ausgelesen werden, beispielsweise über ein digitales Bussystem. Die Separierung des Versorgungssignals in diesem Sinne ist so zu verstehen, dass die einzelnen Vorschaltgeräte der verschiedenen Leuchtkörper durchaus in einem gemeinsamen Gehäuse oder auf einer Platine integriert sein können. Wesentlich ist nur die Separierung der Versorgungssignale der Leuchtkörper.

Die Aufgabe der Erfindung wird für eine eingangs genannte Vorrichtung durch die kennzeichnenden Merkmale des Anspruchs 19 gelöst. Durch die Implementierung des Verfahrens nach einem der Ansprüche 1 bis 18 auf einem Prozessrechner einer Vorrichtung zur Desinfektion einer Substanz kann zum einen die Lebensdauer der einzelnen Leuchtkörper optimal ausgenutzt und zum anderen die optimale Funktion der Vorrichtung sichergestellt werden.

In bevorzugter Ausführung wird durch die ultraviolette Strahlung der Leuchtkörper Ozon erzeugt, wobei die Desinfektion der Substanz zumindest teilweise durch das erzeugte Ozon erfolgt. Bei besonders geeigneten Vorrichtungen ist die Substanz Wasser, insbesondere Abwasser oder Trinkwasser. Bei solchen Anlagen stehen die Leuchtkörper regelmäßig unter erheblicher thermischer Beeinflussung durch das Wasser. Änderung der Wassertemperatur oder auch Fließgeschwindigkeit beeinflussen die Leuchtkörper insbesondere im Fall von Gasentladungsröhren so deutlich, dass ein Vergleich von aktuellen Betriebsparametern mit festen Sollwerten nicht zuverlässig ist. Somit erzielt die dynamische Ermittlung von Referenzwerten gemäß dem erfindungsgemäßen und in die Vorrichtung implementierten Verfahren besonders gute Ergebnisse.

In einer besonders vorteilhaften Ausführung sind die Parameter der Leuchtkörper über ein Bussystem des zumindest einen Vorschaltgerätes auslesbar. Dies ermöglicht eine kostengünstige und einfache Kombination üblicher Vorschaltgeräte mit einem Prozessrechner.

Weitere Vorteile und Merkmale der Erfindung ergeben sich aus dem nachfolgend beschriebenen Ausführungsbeispiel sowie aus den abhängigen Ansprüchen.

Nachfolgend werden zwei bevorzugte Ausführungsbeispiele der erfindungsgemäßen Vorrichtung beschrieben und anhand der anliegenden Zeichnungen näher erläutert.
- Fig. 1: zeigt eine schematische Darstellung eines ersten Ausführungsbeispiels einer erfindungsgemäßen Vorrichtung.
- Fig. 2: zeigt eine schematische Darstellung eines zweiten Ausführungsbeispiels einer erfindungsgemäßen Vorrichtung.

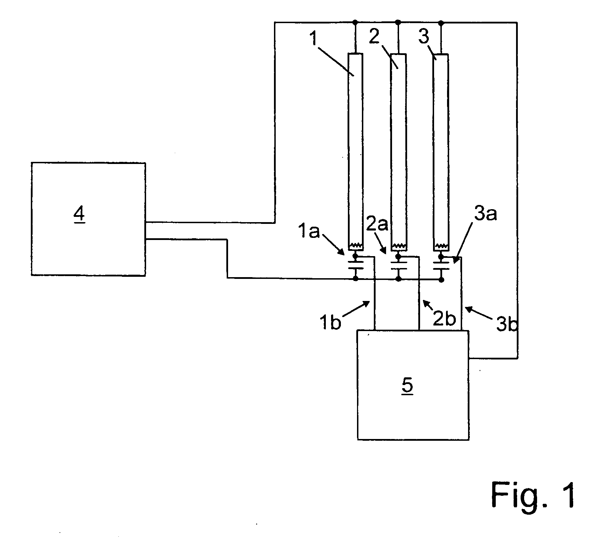

Das erste Ausführungsbeispiel gemäß Fig. 1 umfasst drei UV-Gasentladungsröhren 1, 2, 3, die über ein gemeinsames Vorschaltgerät 4 betrieben werden. Jeder der Röhren 1, 2, 3 ist ein Kondensator 1a, 2a, 3a in Serie vorgeschaltet. Von jeder der Röhren 1, 2, 3 zweigt eine separate Messleitung 1b, 2b, 3b von ihrer kondensatorseitigen Kontaktierung ab und führt zu in einem Prozessrechner 5. Hierdurch kann jede individuelle Versorgungsspannung der drei Röhren von dem Prozessrechner 5 ausgelesen werden, wozu der Prozessrechner 5 ein entsprechendes Messinterface umfasst.

Das Vorschaltgerät 4 gibt als Versorgungsspannung eine rechteckige Wechselspannung von variablen Tastverhältnis aus. Eine effektive Versorgungsspannung ist als zeitlicher Mittelwert des Betrages der Wechselspannung definiert, so ist über das Tastverhältnis die Effektivspannung einstellbar ist.

Ein effektiver, d. h. zeitlich gemittelter Strom wird über das Vorschaltgerät auf einem konstanten Sollwert gehalten, wobei das Tastverhältnis der Spannung die geregelte Größe ist. Hat z. B. jede der baugleichen Gasentladungsröhren 1, 2, 3 einen nominellen Betriebsstrom von 1 Ampere, so regelt das Vorschaltgerät auf einen konstanten Gesamtstrom von 3 Ampere.

Bei normalem Zustand der drei Röhren ergeben sich somit zu jedem Zeitpunkt weitgehend gleiche Betriebsspannungen für jede der einzelnen Röhren. Falls eine der Röhren sich dem Ende ihrer Lebensdauer nähert, so weicht ihr Betriebsspannungswert, der über eine der Leitungen 1b, 2b, 3b ausgelesen wird, von dem Betriebsspannungswerten der jeweils beiden anderen Röhren ab. Der Prozessrechner 5 liest in zeitlichen Abständen, die erheblich kleiner als die Betriebsdauer der Röhren sind (z. B. einmal pro Minute) die effektiven Betriebsspannungen der drei Röhren aus. Wichtig ist, dass die drei Betriebsspannungen im wesentlichen zeitgleich ausgelesen werden, um Einflüsse von sich ändernden Umweltparametern auszuschließen. Die drei Röhren 1, 2, 3 der Vorrichtung aus Fig. 1 sind in einer Wasser-Desinfektionsanlage angeordnet und werden ständig von Wasser umströmt, welches seine Strömungsgeschwindigkeit oder Temperatur ändern kann.

Nach dem Auslesen eines Tripels von Effektivspannungen als Parameter der drei Röhren 1, 2, 3 wird ein Mittelwert aus den drei Spannungswerten gebildet. Nachfolgend wird durch den Prozessrechner dieser Mittelwert mit jeder einzelnen der drei Spannungen verglichen. Weicht eine der drei Spannungen um mehr als 5 % von dem Mittelwert bzw. Referenzwert ab, so generiert der Prozessrechner 5 ein Signal, das die Auffälligkeit der betreffenden Röhre anzeigt.

**Tabelle 1**

| | U_{eff1} [V] | U_{eff2} [V] | U_{eff3} [V] |
|---|---|---|---|
| T_{Wasser} = 5°C | 90, 7 | 92,5 | 92,4 |
| T_{Wasser} = 30°C | 93,1 | 99,8 | 95,1 |

Tabelle 1 zeigt ein konkretes Beispiel von Messwerten von drei Gasentladungsröhren. Es wird jeweils auf konstanten Strom geregelt, wobei die drei Strahler oder Röhren 1, 2, 3 jeweils gemessene Effektivspannungen U_{eff1}, U_{eff2}, U_{eff3} aufweisen. In diesem Beispiel deutet sich eine zu geringe Spannung der ersten Röhre 1 an, die um knapp 2% unter der mittleren Spannung der beiden anderen Röhren liegt. Die Messwerte wurden jeweils bei einer wassertemperatur von 5°C und von 30°C aufgenommen. Ersichtlich wird durch diesen Temperaturunterschied eine ähnlich große Änderung der Spannungswerte bewirkt wie es der Abweichung der Röhre 1 von den anderen Röhren 2, 3 entspricht.

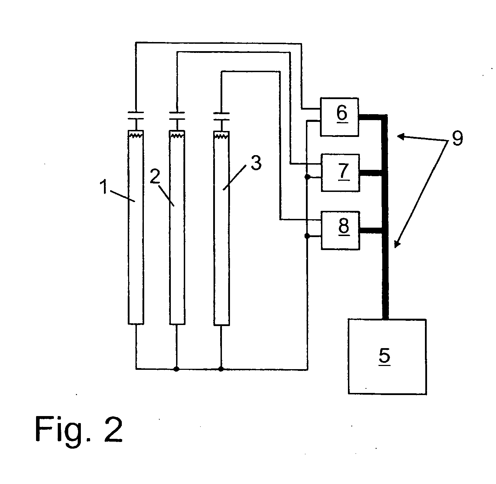

Bei dem zweiten Ausführungsbeispiel gemäß Fig. 2 sind den drei Röhren 1, 2, 3 jeweils individuelle Vorschaltgeräte 6, 7, 8 zugeordnet. Jedes der Vorschaltgeräte 6, 7, 8 regelt jede der drei Röhren 1, 2, 3 separat auf den gleichen konstanten Strom, wobei wie im ersten Ausführungsbeispiel die Spannung eine Rechteck-Wechselspannung mit variablem Tastverhältnis ist. Die Vorschaltgeräte 6, 7, 8 sind mit Hilfe von Digitalelektronik aufgebaut und verfügen über ein Bussystem 9, über das sie verschaltet sind und angesteuert werden können. Ein Prozessrechner 5 liest über das Bussystem 9 die für die jeweiligen Röhren 1, 2, 3 eingeregelten effektiven Versorgungsspannungen unmittelbar aus den Vorschaltgeräten 6, 7, 8 aus, ohne dass zusätzliche Messleitungen an den Röhren erforderlich sind. Diese Werte werden wie zuvor beschrieben zur Bildung eines dynamischen Referenzwertes herangezogen, wobei eine Überschreitung einer Abweichung von 5% der Effektivspannung von dem Referenzwert zur Generierung eines Signals für die jeweils betroffene Röhre führt.

Das erfindungsgemäße Verfahren und die erfindungsgemäße Vorrichtung sind insbesondere für drei oder mehr baugleiche Röhren geeignet. In den schematischen Ausführungsbeispielen wurden lediglich drei Röhren dargestellt; im allgemeinen werden jedoch deutlich mehr Röhren Verwendung finden, insbesondere in großen Anlagen zur Desinfizierung von Trinkwasser und/oder Abwasser.

## Patentansprüche

1. Verfahren zur Überwachung einer Mehrzahl von zumindest drei baugleichen elektrischen Leuchtkörpern (1, 2, 3), umfassend
a. Beaufschlagung der Mehrzahl der Leuchtkörper (1, 2, 3) mit zumindest einem Versorgungssignal aus zumindest einem Vorschaltgerät (4, 6, 7, 8),
b. Auslesen zumindest eines Parameters für jeweils jeden einzelnen Leuchtkörper (1, 2, 3),
c. Bilden zumindest eines Referenzwertes aus zumindest einigen der ausgelesenen Parameter der verschiedenen Leuchtkörper (1, 2, 3),
d. Vergleich des Referenzwertes mit dem Parameter eines einzelnen der Leuchtkörper (1, 2, 3),
e. Generierung eines Signals für jeden Leuchtkörper (1, 2, 3), dessen Parameter eine vorgegebene Abweichung von dem Referenzwert überschreitet.

2. Verfahren nach Anspruch 1, **dadurch gekennzeichnet, dass** der Referenzwert ein Mittelwert der Parameter ist.

3. Verfahren nach Anspruch 1 oder 2, **dadurch gekennzeichnet, dass** der Referenzwert aus sämtlichen der ausgelesenen Parameter gebildet wird.

4. Verfahren nach Anspruch 1 oder 2, **dadurch gekennzeichnet, dass** der Referenzwert jeweils ohne Einbeziehung des in Schritt c. mit dem Referenzwert zu vergleichenden Parameters gebildet wird.

5. Verfahren nach einem der vorhergehenden Ansprüche, **dadurch gekennzeichnet, dass** die Schritte b. bis e. insbesondere in gleichen Zeitabständen wiederholt werden.

6. Verfahren nach Anspruch 5, **dadurch gekennzeichnet, dass** der Parameter für jeden der Leuchtkörper (1, 2, 3) über einen Zeitraum mehrfach ausgelesen und gespeichert wird, wobei ein zeitlicher Verlauf der Parameter und des Referenzwerts ermittelbar ist.

7. Verfahren nach einem der vorhergehenden Ansprüche, **dadurch gekennzeichnet, dass** ein Signal für einen Leuchtkörper generiert wird, wenn eine zeitliche Änderung der Abweichung seines Parameters von dem Referenzwert einen festgelegten Grenzwert überschreitet.

8. Verfahren nach einem der vorhergehenden Ansprüche, **dadurch gekennzeichnet, dass** das Versorgungssignal des Vorschaltgerätes (4, 6, 7, 8) in Abhängigkeit von einem Sollwert geregelt ist.

9. Verfahren nach Anspruch 8, **dadurch gekennzeichnet, dass** der Parameter eines Leuchtkörpers (1, 2, 3) aus dem an ihm anliegenden Versorgungssignal ermittelt wird.

10. Verfahren nach Anspruch 9, **dadurch gekennzeichnet, dass** der Parameter eine effektive Versorgungsspannung (U_{eff1}, U_{eff2}, U_{eff3}) des Leuchtkörpers (1, 2, 3) ist.

11. Verfahren nach Anspruch 10, **dadurch gekennzeichnet, dass** der Referenzwert ein Mittelwert der Parameter der Leuchtkörper ist, wobei die vorgegebene Abweichung nicht mehr als etwa 10%, insbesondere etwa 5% von dem Mittelwert ist.

12. Verfahren nach einem der Ansprüche 8 bis 11, **dadurch gekennzeichnet, dass** der Sollwert eine effektive Stromstärke eines oder mehrerer Leuchtkörper (1, 2, 3) ist.

13. Verfahren nach einem der Ansprüche 8 bis 11, **dadurch gekennzeichnet, dass** der Sollwert eine elektrische Leistung eines oder mehrerer Leuchtkörper (1, 2, 3) ist.

14. Verfahren nach einem der Ansprüche 8 bis 13, **dadurch gekennzeichnet, dass** das Versorgungssignal über eine Pulsweitenmodulation geregelt ist.

15. Verfahren nach Anspruch 14, **dadurch gekennzeichnet, dass** über ein Tastverhältnis des Versorgungssignals eine effektive Versorgungsspannung einstellbar ist, wobei das Versorgungssignal insbesondere eine Rechteckspannung von konstanter Amplitude ist.

16. Verfahren nach einem der vorhergehenden Ansprüche, **dadurch gekennzeichnet, dass** die Leuchtkörper (1, 2, 3) jeweils Gasentladungsröhren sind.

17. Verfahren nach einem der vorhergehenden Ansprüche, **dadurch gekennzeichnet, dass** die Mehrzahl von Leuchtkörpern zumindest zehn, insbesondere zumindest einhundert Leuchtkörper umfasst.

18. Verfahren nach einem der vorhergehenden Ansprüche, **dadurch gekennzeichnet, dass** jedem der Leuchtkörper (1, 2, 3) ein Vorschaltgerät (6, 7, 8) im Sinn eines unabhängigen Versorgungssignals zugeordnet ist.

19. Vorrichtung zur Desinfektion einer Substanz mittels ultravioletter Strahlung, umfassend
eine Mehrzahl von Leuchtkörpern (1, 2, 3), insbesondere Gasentladungsröhren, zur Erzeugung ultravioletter Strahlung,
zumindest ein Vorschaltgerät (4, 6, 7, 8) zur Beaufschlagung der Leuchtkörper (1, 2, 3) mit einem Versorgungssignal, und
ein Prozessrechner (5) mit einem darauf programmierten Steuerprogramm,
wobei das Steuerprogramm ein Verfahren zur Überwachung der Mehrzahl von Leuchtkörpern nach einem der Ansprüche 1 bis 18 umfasst.

20. Vorrichtung nach Anspruch 19, **dadurch gekennzeichnet, dass** durch die ultraviolette Strahlung Ozon erzeugt wird, wobei die Desinfektion der Substanz zumindest teilweise durch das erzeugte Ozon erfolgt.

21. Vorrichtung nach Anspruch 19 oder 20, **dadurch gekennzeichnet, dass** die Substanz Wasser, insbesondere Abwasser oder Trinkwasser, ist.

22. Vorrichtung nach Anspruch 21, **dadurch gekennzeichnet, dass** das Wasser in thermischem Kontakt mit den Leuchtkörpern (1, 2, 3) steht.

23. Vorrichtung nach einem der Ansprüche 19 bis 22, **dadurch gekennzeichnet, dass** die Leuchtkörper (1, 2, 3) Gasentladungsröhren sind.

24. Vorrichtung nach einem der Ansprüche 19 bis 23, **dadurch gekennzeichnet, dass** die Parametern der Leuchtkörper über ein Bussystem (9) des zumindest einen Vorschaltgeräts (6, 7, 8) auslesbar sind.

## Claims

1. Method for monitoring a plurality of at least three electrical illuminants (1, 2, 3) of the same structural design, comprising
a. imposition onto the plurality of illuminants (1, 2, 3) of at least one supply signal from at least one ballast (4, 6, 7, 8),
b. reading out of at least one parameter for each individual illuminant (1, 2, 3) respectively,
c. formation of at least one reference value from at least some of the parameters read out from the various illuminants (1, 2, 3),
d. comparison of the reference value with the parameter of an individual one of the illuminants (1, 2, 3),
e. generation of a signal for each illuminant (1, 2, 3) of which the parameter exceeds a specified deviation from the reference value.

2. Method according to claim 1, **characterised in that** the reference value is a mean value of the parameters.

3. Method according to claim 1 or 2, **characterised in that** the reference value is formed from all the parameters read out.

4. Method according to claim 1 or 2, **characterised in that** the reference value is formed in each case without the involvement of the parameter to be compared with the reference value in step c.

5. Method according to any one of the preceding claims, **characterised in that** the steps b. to e. are repeated in particular at equal time intervals.

6. Method according to claim 5, **characterised in that** the parameter for each of the illuminants (1, 2, 3) is read out and stored repeatedly over a period, wherein a temporal course of the parameters and of the reference value can be determined.

7. Method according to any one of the preceding claims, **characterised in that** a signal for an illuminant is generated if a temporal change in the deviation of its parameter from the reference value exceeds a determined limit value.

8. Method according to any one of the preceding claims, **characterised in that** the supply signal from the ballast (4, 6, 7, 8) is regulated as a function of a desired value.

9. Method according to claim 8, **characterised in that** the parameter of an illuminant (1, 2, 3) is determined from the supply signal applied to it.

10. Method according to claim 9, **characterised in that** the parameter is an effective supply voltage (U_{eff1}, U_{eff2}, U_{eff3}) of the illuminant (1, 2, 3).

11. Method according to claim 10, **characterised in that** the reference value is a mean value of the parameters of the illuminants, wherein the specified deviation is not more than approximately 10%, in particular approximately 5%, of the mean value.

12. Method according to any one of claims 8 to 11, **characterised in that** the reference value is an effective current strength of one or more illuminants (1, 2, 3).

13. Method according to any one of claims 8 to 11, **characterised in that** the desired value is an electrical output of one or more illuminants (1, 2, 3).

14. Method according to any one of claims 8 to 13, **characterised in that** the supply signal is regulated by means of a pulse width modulation.

15. Method according to claim 14, **characterised in that**, by means of a pulse duty factor of the supply signal, an effective supply voltage can be adjusted, wherein the supply signal is in particular a square-wave voltage of constant amplitude.

16. Method according to any one of the preceding claims, **characterised in that** the illuminants (1, 2, 3) are in each case gas discharge tubes.

17. Method according to any one of the preceding claims, **characterised in that** the plurality of illuminants comprise at least ten, and in particular at least one hundred, illuminants.

18. Method according to any one of the preceding claims, **characterised in that** a ballast (6, 7, 8) in the sense of an independent supply signal is allocated to each of the illuminants (1, 2, 3).

19. Device for disinfecting a substance by means of ultraviolet radiation, comprising
a plurality of illuminants (1, 2, 3), in particular gas discharge tubes, for generating ultraviolet radiation,
at least one ballast (4, 6, 7, 8) for imposing a supply signal on the illuminants (1, 2, 3), and
a process computer (5) with a control program programmed into it,
wherein the control program comprises a method for monitoring the plurality of illuminants in accordance with any one of claims 1 to 18.

20. Device according to claim 19, **characterised in that** ozone is produced by the ultraviolet radiation, wherein the disinfection of the substance is effected at least partially by the ozone produced.

21. Device according to claim 19 or 20, **characterised in that** the substance is water, in particular waste water or drinking water.

22. Device according to claim 21, **characterised in that** the water is in thermal contact with the illuminants (1, 2, 3).

23. Device according to any one of claims 19 to 22, **characterised in that** the illuminants (1, 2, 3) are gas discharge tubes.

24. Device according to any one of claims 19 to 23, **characterised in that** the parameters of the illuminants can be read off by means of a bus system (9) of the at least one ballast (6, 7, 8).

## Revendications

1. Procédé de surveillance d'une pluralité d'au moins trois éléments lumineux électriques de structure semblable (1, 2, 3), comportant
a) l'alimentation de la pluralité d'éléments lumineux (1, 2, 3) par au moins un signal d'alimentation provenant d'au moins un ballast (4, 6, 7, 8),
b) l'extraction d'au moins un paramètre pour chaque élément lumineux particulier respectif (1, 2, 3),
c) la formation d'au moins une valeur de référence à partir d'au moins certains des paramètres extraits des différents éléments lumineux (1, 2, 3),
d) la comparaison de la valeur de référence au paramètre de l'un particulier des éléments lumineux (1, 2, 3),
e) la génération d'un signal pour chaque élément lumineux (1, 2, 3), dont un paramètre dépasse un écart prédéterminé de la valeur de référence.

2. Procédé selon la revendication 1, **caractérisé en ce que** la valeur de référence est une valeur moyenne des paramètres.

3. Procédé selon la revendication 1 ou 2, **caractérisé en ce que** la valeur de référence est formée à partir de l'ensemble des paramètres extraits.

4. Procédé selon la revendication 1 ou 2, **caractérisé en ce que** la valeur de référence est réalisée respectivement sans l'insertion du paramètre à comparer à la valeur de référence au cours de l'étape c).

5. Procécé selon l'une des revendications précédentes, **caractérisé en ce que** les étapes b) à e) sont répétées en particulier pendant des périodes de temps semblables.

6. Procédé selon la revendication 5, **caractérisé en ce que** le paramètre pour chacun des éléments lumineux (1, 2, 3) est extrait plusieurs fois pendant une période et mémorisé, moyennant quoi une courbe temporelle des paramètre et de la valeur de référence peut être déterminée.

7. Procédé selon l'une des revendications précédentes, **caractérisé en ce qu'**un signal pour une élément lumineux est généré, lorsqu'un changement temporel de l'écart de son paramètre dépasse une valeur limite déterminée de la valeur de référence.

8. Procédé selon l'une des revendications précédentes, **caractérisé en ce que** le signal d'alimentation du ballast (4, 6, 7, 8) est réglé selon une valeur de consigne.

9. Procédé selon la revendication 8, **caractérisé en ce que** le paramètre d'un élément lumineux (1, 2, 3) est déterminé à partir d'un signal d'alimentation qui lui est appliqué.

10. Procédé selon la revendication 9, **caractérisé en ce que** le paramètre est une tension d'alimentation effective (U_{eff1}, U_{eff2}, U_{eff3}) de l'élément lumineux (1, 2, 3).

11. Procédé selon la revendication 10, **caractérisé en ce que** la valeur de référence est une valeur moyenne des paramètres des éléments lumineux, moyennant quoi l'écart prédéterminé n'est pas supérieur à environ 14%, en particulier environ 5% de la valeur moyenne.

12. Procédé selon l'une de revendications 8 à 11, **caractérisé en ce que** la valeur de consigne est une intensité de courant effective d'un ou de plusieurs éléments lumineux (1, 2, 3).

13. Procédé selon l'une des revendications 8 à 11, **caractérisé en ce que** la valeur de consigne est une capacité électrique d'un ou de plusieurs éléments lumineux (1, 2, 3).

14. Procédé selon l'une des revendications 8 à 13, **caractérisé en ce que** le signal d'alimentation est réglé au-dessus d'une modulation de largeur d'impulsion.

15. Procédé selon la revendication 14, **caractérisé en ce qu'**une tension d'alimentation effective peut être réglée par l'intermédiaire d'un rapport cyclique du signal d'alimentation, moyennant quoi le signal d'alimentation est en particulier une tension rectangulaire d'amplitude constante.

16. Procédé selon l'une des revendications précédentes, **caractérisé en ce que** les éléments lumineux (1, 2, 3) sont respectivement des tubes à gaz.

17. Procédé selon l'une des revendications précédentes, **caractérisé en ce que** la pluralité des éléments lumineux comprend au moins dix, en particulier au moins onze éléments lumineux.

18. Procédé selon l'une des revendications précédentes, **caractérisé en ce qu'**un ballast (6, 7, 8) est associé à chacun des éléments lumineux (1, 2, 3) dans le sens d'un signal d'alimentation indépendant.

19. Dispositif pour désinfecter une substance au moyen d'un rayonnement ultraviolet, comportant
une pluralité d'éléments lumineux (1, 2, 3), en particulier des tubes à gaz, pour la génération d'un rayonnement ultraviolet,
au moins un ballast (4, 6, 7, 8) pour alimenter les éléments lumineux (1, 2, 3) avec un signal d'alimentation, et
un ordinateur de traitement (5) comportant un programme de commande programmé,
dans lequel le programme de commande comporte un procédé pour surveiller la pluralité d'éléments lumineux selon l'une des revendications 1 à 18.

20. Dispositif selon la revendication 19, **caractérisé en ce que** de l'ozone est généré par le rayonnement ultraviolet, moyennant quoi la désinfection de la substance s'effectue au moins partiellement grâce à l'ozone généré.

21. Dispositif selon la revendication 19, **caractérisé en ce que** la substance est de l'eau, en particulier des eaux usées ou de l'eau potable.

22. Dispositif selon la revendication 21, **caractérisé en ce que** l'eau est en contact thermique avec les éléments lumineux (1, 2, 3).

23. Dispositif selon l'une des revendications 19 à 22, **caractérisé en ce que** les éléments lumineux (1, 2, 3) sont des tubes à gaz.

24. Dispositif selon l'une des revendications 19 à 23, **caractérisé en ce que** les paramètres des éléments lumineux peuvent être extraits par l'intermédiaire d'un système de bus (9) d'au moins un ballast (6, 7, 8).
